Europäisches Patentamt

European Patent Office

Office européen des brevets

(19)

(11) Veröffentlichungsnummer: **0 336 285**
**A2**

(12) **EUROPÄISCHE PATENTANMELDUNG**

(21) Anmeldenummer: 89105581.6

(51) Int. Cl.⁴: **C12N 9/16 , C12N 9/22**

(22) Anmeldetag: 30.03.89

(30) Priorität: 02.04.88 DE 3811279

(43) Veröffentlichungstag der Anmeldung:
11.10.89 Patentblatt 89/41

(84) Benannte Vertragsstaaten:
AT BE CH DE ES FR GB GR IT LI LU NL SE

(71) Anmelder: BOEHRINGER MANNHEIM GMBH
Sandhofer Strasse 116
D-6800 Mannheim 31(DE)

(72) Erfinder: Bolton, Bryan John
7 Chalfont Court Bracken Lane
Shirley Southampton(GB)
Erfinder: Jarsch, Michael, Dr.rer.nat.
Unterkarpfsee 11
D-8173 Bad Heilbrunn(DE)
Erfinder: Schmitz, Gudrun, Dr.rer.nat.
Wettersteinstrasse 3
D-8139 Bernried(DE)
Erfinder: Kessler, Christoph, Dr.rer.nat.
Fraunhoferstrasse 12
D-8000 München 5(DE)

(54) **Typ II-Restriktionsendonuklease AspI.**

(57) Die Restriktionsendonuklease AspI besitzt folgende Erkennungssequenz, spaltet bevorzugt an der durch die Markierung definierten Spaltstelle:

$$5'\text{-GACN}|\text{N-NGTC}$$

$$3'\text{-CTGN-N}|\text{NCAG}$$

und ist aus Mikroorganismen der Gattung Achromobacter erhältlich. Sie ist isoschizomer zu TthI und kann zur Erkennung und Spaltung der komplementären doppelsträngigen DNA-Sequenz 5'-GACNNNGTC-3' eingesetzt werden.

EP 0 336 285 A2

## TYP II-Restriktionsendonuklease AspI

Die Erfindung betrifft die Typ II-Restriktionsendonuklease AspI, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch ist ihre Zugänglichkeit häufig unbefriedigend. In vielen Fällen sind die Mikroorganismen, aus denen diese Restriktionsendonukleasen isoliert werden, schwer zu kultivieren oder pathogen. Ebenso ist die Ausbeute bei großtechnischer Herstellung oft gering.

So ist die Restriktionsendonuklease Tth111I (Gene 47 (1986) 1 -153) nur schwierig von Tth111II zu trennen und die Ausbeute ist gering. Darüber hinaus kann von Nachteil sein, daß Tth111I ein relativ hohes Temperaturoptimum, von ca. 65° C, aufweist.

Der Erfindung liegt daher die Aufgabe zu Grunde, ein Isoschizomeres zu Tth111I zur Verfügung zu stellen, welches einfacher und in höherer Ausbeute herstellbar ist und möglichst ein Aktivitätsoptimum bei physiologischer Temperatur aufweist.

Gelöst wird diese Aufgabe erfindungsgemäß durch eine Typ-II-Restriktionsendonuklease mit der Erkennungssequenz und der durch die Markierung definierten Spaltstelle

$$5'\text{-GACN} | \text{N-NGTC-}3'$$
$$3'\text{-CTGN-N} | \text{NCAG-}5'$$

welche dadurch gekennzeichnet ist, daß sie aus Mikroorganismen der Gattung Achromobacter erhältlich ist.

Hierbei bedeutet N: A, G, C oder T, wobei jeweils G mit C und A mit T im komplementären DNA-Strang korrespondiert.

Die erfindungsgemäße neue Restriktionsendonuklease, die im folgenden als AspI bezeichnet wird, hat ein Temperaturoptimum bei ca. 37° C. Das Enzym besitzt eine gute Aktivität zwischen pH 7,2 und pH 7,8 bei einer Konzentration eines einwertigen Kations bei ca. 50 mmol/l (für NaCl) in 10 mmol/l $MgCl_2$ und 10 mmol/l Mercaptoethanol. Das pH-Optimum liegt bei ca. pH 7,5. AspI ist ein Isoschizomer zu Tth111I.

Die Erkennungssequenz läßt sich durch vollständigen Verdau der DNAs der Viren SV40 und Adeno 2, der Phagen Lambda, phiX174, den Phagenderivaten M13mp8 und den Plasmiden pBR322 und pBR328 bestätigen. Diese DNA-Moleküle werden mit AspI behandelt.

Tabelle 1 zeigt einen Vergleich der experimentell beobachteten Schnittstellenspezifität mit einer computerermittelten Schnittstellenspezifität für ein Enzym, welches die Sequenz GACNNNGTC erkennt.

Tabelle 1

| DNA | Anzahl der experimentell bestimmten Schnittstellen | Anzahl der durch Computeranalyse bestimmte Schnittstellen | Experimentell bestimmte Fragmentlängen | Durch Computeranalyse bestimmte Fragmentlängen | Spaltpositionen ermittelt durch Computeranalyse |
|---|---|---|---|---|---|
| SV40 | 0 | 0 | 0 | 0 | 0 |
| phiX174 | 0 | 0 | 0 | 0 | 0 |
| M13mp8 | 0 | 0 | 0 | 0 | 0 |
| pBR322 | 1 | 1 | 4350 bp | 4363 bp | bei 2222 bp |
| pBR328 | 0 | 0 | 0 | 0 | 0 |
| bp: Basenpaar(e) | | | | | |

EP 0 336 285 A2

Die Schnittstelle innerhalb der Erkennungssequenz des Enzyms läßt sich wie folgt feststellen: Das Plasmid pBR322 wird mit NdeI linearisiert. Das NdeI-Fragment wird am 5-Ende mit T4-Polynucleotid-Kinase unter Verwendung von [γ$^{32}$P]-ATP markiert und am 3'-Ende mit terminaler Transferase und [α$^{32}$P]-ddATP $^{32}$P-markiert. Nach einem weiteren Verdau werden die entstandenen Fragmente, welche entweder am NdeI-Ende 5'-markiert oder 3'-markiert sind, durch Agarosegel-Elektrophorese isoliert und aus dem Gel gereinigt.

Aliquote dieser Fragmente werden mit AspI behandelt und gleichzeitig einer basenspezifischen chemischen Spaltung (Maxam and Gilbert, Methods in Enzymology 65, 1980, 499 - 560) zur Sequenzierung unterworfen.

Die Analyse der Fragmente wird durch Sequenzgel Elektrophorese (6 % Polyacrylamid, 8 mol/l Harnstoff) und anschließende Autoradiographie durchgeführt. Die Interpretation der Ergebnisse wird analog Methods in Enzymology 65, 1980, 391 - 401 durchgeführt. Hierbei zeigt sich, daß AspI zwischen Position 2222 und 2223 (Basenpaare) in der Sequenz des Vektors pBR322 mit folgender Spezifität schneidet:

$$5'\text{ATGACC} \,|\, \text{C-AGTCAC-}3'$$

$$3'\text{TACTGG-G} \,|\, \text{TCAGTG-}5'$$

Damit ergibt sich als allgemeine Spezifität:

$$5'\text{-GACN} \,|\, \text{N-NGTC}$$

$$3'\text{-CTGN-N} \,|\, \text{NCAG}$$

Die experimentell bestimmte Anzahl der Schnittstellen ist identisch mit der Anzahl der Schnittstellen, die durch Computeranalyse mit den verschiedenen DNAs für die Sequenz GACNNNGTC erhalten wurde (Tabelle I). Zusätzlich wurden diese Daten noch mit den Tabellen aus Gene 10 (1980) 357 - 370, verglichen. Ein doppelter Verdau von pBR322 DNA mit PstI und AspI sowie von pBR328 DNA mit PstI und AspI bestätigen, daß die Spezifität GACNNNGTC ist.

Erfindungsgemäß wird AspI gewonnen, indem man Mikroorganismen der Gattung Achromabacter züchtet und das Enzym aus den Zellen gewinnt. Vorzugsweise wird Achromobacter spec. DSM 4318 verwendet. Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Die für die Gewinnung des Enzyms verwendeten Mikroorganismen wachsen aerob in Merck Standard I Medium. Achromobacter spec. wurde bei der Deutschen Sammlung von Mikroorganismen, Gesellschaft für biotechnologische Forschung mbH, Mascheroder Weg 16, 3300 Braunschweig, BRD, hinterlegt und trägt die Hinterlegungsnummer DSM 4318. Die optimalen Wachstumsbedingungen sind bei 10 -30° C, pH 7,2 - 7,8. Die Verdoppelungszeit beträgt etwa 2 Stunden.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen einem Überdruck von 5 bar ausgesetzt. Die hierbei entstandene Zellmasse wird in Tris HCl Puffer, pH 8,0, welcher Protease-Inhibitoren enthält, resuspendiert. Anschließend werden die Zellen über eine French-Presse aufgeschlossen und mit Polymin und Ammoniumsulfat präzipitiert. Die weitere Reinigung des Überstands wird vorzugsweise über Molekularsiebchromatographie, Ionentauscherchromatographie sowie Affinitätschromatographie durchgeführt. Ein Beispiel für ein geeignetes Molekularsieb ist Ultrogel AcA34 (LKB).

Als Anionentauscher geeignet ist das unter dem Namen DEAE Sepharose Fast Flow (Pharmacia) erhältliche Produkt. Als Material für die Affinitätschromatographie geeignet ist beispielsweise Heparin-Sepharose CL-6B (Pharmacia). Aber auch andere Chromatographiematerialien, die dem Fachmann bekannt sind, sind geeignet.

Die folgenden Beispiele erläutern die Erfindung weiter.

Beispiel 1

Achromobacter spec. DSM 4318 wird bei 30°C 20 Stunden gezüchtet und in der späten logarithmischen bzw. stationären Phase geerntet. Als Kulturmedium wird Merck Standardmedium I verwendet.

Die Zellpaste (30 g Naßgewicht) wird in 3 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch² aufgeschlossen. Anschließend wird Polymin P zugegeben und der Niederschlag abgetrennt. Der Überstand wird anschließend durch Zugabe von 50 % (w/v) Ammoniumsulfat behandelt. Der hierbei entstandene Niederschlag wird in 15 ml Puffer B (40 mmol/l Tris-HCl, pH 8,0, 0,1 mmol/l EDTA, 7 mmol/l 2-Mercaptoethanol, 10 % (V/V) Glyclerin) aufgelöst. Anschließend wird nach Dialyse gegen Puffer B an eine Heparin-Sepharose-Säule fraktioniert. Zur Elution wird ein Gradient von 0 - 1 M NaCl verwendet. AspI wird in den Fraktionen zwischen 0,2 und 0,3 M NaCl gefunden. Die aktiven Fraktionen werden mit 90 % Ammoniumsulfat behandelt und der Niederschlag nach Dialyse gegen Puffer B wird an einer Ultrogel AcA34-Säule fraktioniert, welche zuvor mit Puffer B, dem 0,5 mmol/l NaCl zugesetzt wird, equilibriert ist. Die aktive Fraktion wird gegen Puffer B dialysiert. Anschließend wird sie auf eine DEAE Sepharose Fast Flow-Säule, welche mit Puffer B equilibriert wurde, aufgegeben. Zur Elution wird ein Gradient von 0 - 1,0 mmol/l NaCl in Puffer B verwendet.

Die aktiven Fraktionen der Fraktionen werden zusammengegeben und gegen Lagerpuffer (20 mmol/l Tris-HCl, pH 8,0, 10 mmol/l 2-Mercaptoethanol und 100 mmol/l NaCl, 50% (v/v) Glycerin) dialysiert.

Beispiel 2

Bestimmung der Aktivität

Definition der Enzymeinheiten:

1 U AspI spaltet 1 µg Lambda-DNA innerhalb 1 Stunde bei 37°C in 25 µl Endvolumen.

Zu einer Mischung von 2,5 µl Inkubationspuffer (80 mmol/l Tris-HCl, pH 7,5/37°C, 100 mmol/l Magnesiumchlorid, 500 mmol/l NaCl und 100 mmol/l 2-Mercaptoethanol) 100 µg/ml RSA (Rinderserumalbumin) werden 1,5 µl Wasser und 5 µl Lambda DNA (optische Dichte: 4 OD/ml) sowie 1 µl AspI-Lösung (1 U/µl) zugegeben. Die Lösung wird eine Stunde bei 37°C inkubiert, auf Eis gekühlt und mit 5µl eines Abstopp-Reagenses, bestehend aus 7 mmol/l Harnstoff, 20 % (w/v) Saccharose, 60 mmol/l EDTA und 0,01 % (w/v) Bromphenolblau versetzt. Anschließend wird eine Trennung durch Elektrophorese in 0,8 % Agarose-Gelen für 3 - 4 Stunden bei 100 V durchgeführt. Die erhaltenen Banden werden über Vergleich mit einem DNA-Längenstandard identifiziert.

## Ansprüche

1. Typ II-Restriktionsendonuklease mit der Erkennungssequenz und der durch die Markierung charakterisierten Schnittstelle

$$5' - GACN \mid N - NGTC - 3'$$

$$3' - CTGN - N \mid NCAG - 5'$$

dadurch gekennzeichnet, daß sie ein Temperaturoptimum von ca. 37°C aufweist und aus Mikroorganismen der Gattung Achromobacter erhältlich ist.

2. Restriktionsendonuklease nach Anspruch 1 dadurch-gekennzeichnet, daß sie aus Achromobacter spec. DSM 4318 erhalten wird.

3. Restriktionsendonuklease nach den Ansprüchen 1 und 2, gekennzeichnet durch ein pH-Optimum zwischen 7,2 und 7,8.

4. Verfahren zur Gewinnung einer Restriktionsendonuklease mit der Erkennungssequenz und der durch die Markierung charakterisierten Spaltstelle

$$5' - \text{GACN} | \text{N} - \text{NGTC} - 3'$$

$$3' - \text{CTGN} - \text{N} | \text{NCAG} - 5'$$

dadurch gekennzeichnet, daß man Mikroorganismen der Gattung Achromobacter züchtet und das Enzym aus den Zellen gewinnt.

5. Verfahren nach Anspruch 4, dadurch gekennzeichnet, daß man Achromobacter spec. DSM 4318 züchtet.

6. Verfahren nach den Ansprüchen 4 und 5, dadurch gekennzeichnet, daß man die Zellen aufschließt und den Zellüberstand gewinnt.

7. Verfahren nach Anspruch 6, dadurch gekennzeichnet, daß man zur Feinreinigung den Zellüberstand einer Affinitätschromatographie, einer Molekularsiebchromatographie und einer abschließenden Chromatographie über einen Anionentauscher unterwirft.

8. Verfahren nach Anspruch 7, dadurch gekennzeichnet, daß man zur Affinitätschromatographie trägerfixiertes Heparin verwendet.

9. Verwendung einer TypII-Restriktionsendonuklease mit der Erkennungssequenz und der durch die Markierung charakterisierten Schnittstelle

$$5' - \text{GACN} | \text{N} - \text{NGTC} - 3'$$

$$3' - \text{CTGN} - \text{N} | \text{NCAG} - 5' ,$$

die ein Temperaturoptimum von ca. 37° C aufweist und aus Mikroorganismen der Gattung Achromobacter erhäitlich ist, zur Erkennung und Spaltung der komplementären doppelsträngigen DNA-Sequenz 5'-GACNNNGTC-3'.